# EUROPEAN PATENT SPECIFICATION

(11) **EP 3 936 104 B1**
(45) Date of publication and mention of the grant of the patent: **22.03.2023**
(21) Application number: 21178133.1
(22) Date of filing: 08.06.2021
(51) Int. Cl.: A61H 1/02, A61H 1/00

(54) **JOINT WITH TWO DEGREES OF FREEDOM FOR USE IN EXOSKELETONS AND REHABILITATION FACILITIES**
GELENK MIT ZWEI FREIHEITSGRADEN ZUR VERWENDUNG IN EXOSKELETTEN UND REHABILITATIONSEINRICHTUNGEN
ARTICULATION À DEUX DEGRÉS DE LIBERTÉ POUR UTILISATION DANS LES EXOSQUELETTES ET LES ÉTABLISSEMENTS DE RÉÉDUCATION

(30) Priority: 28.06.2020 CZ 20200377
(43) Date of publication of application: 12.01.2022
(73) Proprietor: Vysoka skola banska - Technicka univerzita Ostrava, 70800 Ostrava, Poruba (CZ); Theosun technologies s.r.o., 71000 Ostrava (CZ)
(72) Inventor: GRUSHKO, Stefan, 70800 Ostrava (CZ); STREJCEK, Radim, 71000 Ostrava (CZ)
(74) Representative: Dadej, Leopold

(56) References cited:
- WO-A1-2008/155286
- WO-A1-2012/042471
- CN-A- 110 897 833

## Description

### Field of the Invention

The present invention relates to a field of robotic rehabilitation devices. Specifically, it is a hollow mechanical joint with two degrees of freedom for use in exoskeletons and rehabilitation devices.

### Background of the Invention

Medical robotic exoskeletons are medical means intended for use for humans to mitigate or compensate for injury or medical disability, which is, for example, severe to complete loss of movement of upper or lower limbs. They are often used as rehabilitation means. An important feature of exoskeletons is that they are able to perform the intended movement perfectly, in a controlled and repeated manner. Mechanical joints of exoskeleton must correspond anatomically to joints of the rehabilitated part of the body.

The vast majority of the existing exoskeletons do not address a wrist joint, or they address it only by means of a joint with a single degree of freedom. A disadvantage of these solutions is the impossibility of the joint to bend in one of natural movement axes. Those solutions are disclosed in e.g., patent documents CN106994087, CN104097213 or CN102895091

From the state of the art, e.g., from patent documents CN106473896, US2011282253, WO2015084177 and US2015223959, joint solutions of exoskeletons and rehabilitation devices allowing for movement of the joint in two and more rotation axes are known. However, these solutions do not mechanically ensure stable joint rotation axis, i.e., during the simultaneous activation of drives providing the joint movement in axes, a shift of the mechanism rotation axis outside the user's joint rotation axis may occur, which may result in user's injury. In addition, such mechanical design of the joint does not allow for adding mechanical stoppers which would protect the user's joint against excessive bending. The solution disclosed in a patent document US2006128479 also does not allow the use of the joint for human joint rehabilitation, since in this case the rotation axes do not intersect in a single point.

More sophisticated solutions for exoskeletons, e.g., known from patent documents CN106038169, CN105252522 or US2012179075, for a joint designed with two degrees of freedom with the intersection of the axes corresponding to the actual rotation point of the user's wrist joint, use two ordinary rotation joints. This solution has, depending on the specific mechanical design, a disadvantage of limiting maximum joint rotation in one of the axes - palmar and ulnar duction or palmar and dorsal flexion.

In addition, there are static rehabilitation devices for the wrist joint providing one or more rotation axes, but their construction cannot be, in principle, reduced into compact mobile dimensions. An example of such embodiment is disclosed in a patent document US2015359697.

Other existing solutions of rehabilitation devices and exoskeletons providing a stable rotation axis of a mechanical joint, identical to the user's joint rotation axis, are known from, e.g., patent documents CN101450484, CN106112987 or CN202537871 (U). A disadvantage of these solutions is the structural complexity of member / draw bar mechanisms and large dimensions thereof.

The existing hollow cardan joints are not suitable for use in rehabilitation devices due to the small inner space of the joint for the user's hand. An example of such joint is e.g., a solution known from EP0007318.

In exoskeletons and rehabilitation devices, a rail mechanism is also used to provide stable rotation axis of the mechanical joint identical to the user's joint rotation axis. However, all solutions, disclosed in e.g., patent documents CN105726263, CN204147236 and CN106264983, use the rail mechanism to provide rotation only in a single axis and never for application for the wrist joint.

The solution in a patent document CN106031669 uses a gear rack to provide two rotation axes in an arm, but the construction differs in principle.

The solution in a patent document CN106491196 uses combination of two circular rails for the wrist joint, however, the relative position of the rails does not allow for their minimization to achieve a compact solution. Minimalization would cause a collision with the wrist or limitation of movement.

Patent document WO2012042471 is disclosing a joint with two degrees of freedom for use in exoskeletons and rehabilitation devices for connection of a first and a second relatively moving parts of the exoskeleton.

None of the currently known solutions thus provides an exoskeleton joint suitable for human wrist or other human joints that would concurrently remove the above-mentioned disadvantages.

### Summary of the Invention

The aim of the invention is to provide a compact hollow joint for use in exoskeletons and rehabilitation devices providing wide range of movement comparable to flexibility of a joint on a human limb, simultaneously in two axes and providing that the joint rotation axes correspond to the user's joint rotation axes.

The said aim is achieved by means of a joint with two degrees of freedom for use in exoskeletons and rehabilitation devices to connect first and second relative moving parts of the exoskeleton, the joint comprises at least a first and a second rail in shape of circular arcs which are located in two relative perpendicular planes, wherein the centers of circles of circular arcs differ, and further on each rail a movably arranged slider, essence of which lies in the fact that either the rails are firmly interconnected and each slider is adapted to be connected to a different relatively moving part of the exoskeleton, or the sliders are firmly interconnected and the rails are each adapted to be connected to a different relatively moving part of the exoskeleton.

Straight lines intersecting the centers of circles of the circular arcs and perpendicular to respective planes defined by circular arcs essentially form joint rotation axes around which sliders movable on the rails move. Providing the same place of rotation of the user's joint and the joint according to the invention is necessary to ensure the safe use, e.g., during user's joint rehabilitation. From this point of view, it is ideal if the joint with two degrees of freedom is essentially custom built and fixed to the user, so that the joint rotation axes according to the invention corresponded to the user's joint rotation axes during use. This ensures that rotational movement of relatively moving parts of the exoskeleton can be identical to movements of the user's body parts, i.e., there is no joint damage caused by "stretching" or dislocating thereof.

On the other hand, the majority of joints in human body are more or less spherical, therefore, also with regard to potential series production, the joint according to the invention can ideally be spherical in such way that the straight lines intersecting the centers of circles of the circular arcs and perpendicular to the respective planes defined by circular arcs intersect each other in a single point. In another embodiment, the straight lines intersecting the centers of circles of the circular arcs and perpendicular to the respective planes defined by circular arcs do not need to intersect each other in a single point but can be slightly spaced apart, when in practice the distance of these straight lines is usually lower than the radius of the largest rail.

The center of the circle of one of the circular arcs may be located on a straight line intersecting the center of the circle of the second of the circular arcs, but the centers of circles of the circular arcs cannot be identical.

The subject matter of the invention is based on the use of a double arc rail guide, which has a large inner space and allows, in this embodiment, movement in two axes with a wide range. Thus, at least one rail and a slider moving thereon belongs to each degree of freedom. It does not matter what will be the profile of the used rails.

The invention offers two basic alternatives. In the first case the rails are firmly interconnected and sliders, each adapted to be connected to the first and the second relatively moving parts of the exoskeleton, move relatively to each other along paths defined by the rails. In the second case the sliders are firmly interconnected and rails, each adapted to be connected to the first and the second relatively moving parts of the exoskeleton, move relatively to each other along paths defined by the sliders. Both variants, in comparison with the state of the art, allow to make the joint more compact and at the same time to maintain the biggest range of movement, wherein when two pairs of rails are used, described below, and particularly with the variant with mutually firmly interconnected rails it is possible to ensure maximum rigidity of the design (the best force distribution).

Therefore, either rails or sliders are each adapted to be connected to the first and the second relatively moving parts of the exoskeleton, when the first and the second parts of the exoskeleton serves for attaching on human body parts, the relative safe movement of which should be provided by the joint. The connection of rails or sliders with first and second relatively moving parts of the exoskeleton must be firm, which ensures the highest stability of joint axis. In order to keep coaxiality of the exoskeleton with the human body joint, the first and the second relatively moving parts of the exoskeleton must be adapted so that their displacement relative to the respective human body part is prevented. Thus, the first and the second relatively moving parts of the exoskeleton may be in the form of e.g., adjustable sleeve, glove, etc., with inner surface thereof abutting the respective body parts preferably made of biocompatible/bioinert material.

For example, in a case of a human limb joint, specifically a wrist joint, the first part of the exoskeleton can be connected before the wrist joint at the limb end and physically it can be considered as a sort of a glove, while the second part of the exoskeleton is attached behind the wrist joint of corresponding the limb, i.e., on the forearm, e.g., by means of an adjustable sleeve. The connection of the first and the second parts of the exoskeleton is then provided by the joint according to this invention, which is arranged in between the first and the second parts of the exoskeleton, firmly connected therewith and formed by a set of shaped rails and sliders according to the invention moving thereon.

The joint according to this invention is mechanical and hollow. The radii of circles of circular arcs of rails do not need to be identical. The smaller the radii of these circles, the smaller the joint and the smaller the space inside the joint. Therefore, the choice of the circle radii will depend on the specific application of the joint. The embodiments of radii of the circles will correspond to the sliders.

Central angles of the rail circular arcs define the working range of the joint. The bigger the central angle, wherein with some joints, e.g., shoulder joint, it may be bigger than 180º, the bigger bending in the respective axis the joint offers. The movement of the slider on the rail for one or both rotation axes may be limited by mechanical stoppers provided on the rail. During actual use of the joint in the exoskeleton, it is therefore possible to provide a protection of the human body joint against injury caused by excessive bending, against collision with design parts of the joint according to this invention, or against derailing of the slider.

Individual joint components according to this invention are ideally rigid, as are the first and/or the second relatively moving parts of the exoskeleton, which come into contact with human body. As a result of this, the rotation axes of the joint according to the invention cannot be displaced relative to the human body joint. However, to achieve minimum comfort of the user, the first and/or the second relatively moving parts of the exoskeleton can be designed as flexible.

The basic preferred embodiment of the joint with two degrees of freedom is based on that it is formed by the first and the second rail in shape of circular arcs that are firmly interconnected and are located in two relative perpendicular planes, wherein the straight lines intersecting the centers of circles of circular arcs and perpendicular to the respective planes defined by circular arcs intersect each other in a single point, the first moving part of the exoskeleton is provided with a slider that moves on the first rail, and the second moving part of the exoskeleton is provided with a slider that moves on the second rail.

In another preferred embodiment the joint according to the invention may comprise, in addition to the first and the second rail in shape of circular arcs, a parallel third and fourth rail, the first and the third rail are located in two planes parallel to each other, wherein the centers of circles of their circular arcs are located on a single straight line perpendicular to the respective planes defined by the circular arcs and the circles of circular arcs have the same radius, and the second and the fourth rail are located in two planes parallel to each other, wherein the centers of circles of their circular arcs are located on a single straight line perpendicular to the respective planes defined by the circular arcs. Each rail, as mentioned above, i.e., the third and the fourth one as well, is provided with another slider which moves thereon. Also in this embodiment, either sliders or rails are firmly interconnected. In this parallel arrangement of paired rails, the inner space of the joint is located between the paired rails. This embodiment of the joint with paired rails ("on both sides of the joint") has an advantage that load on the joint is better distributed to both sides of the mechanism and there is no undesirable torque deforming the rail. Thus, it will be particularly suitable for bigger joints. On the other hand, a joint with unpaired rails allows for easier attachment to the user's joint. In addition, it is more compact and allows for bigger movement range, since the central angles of circular arcs of rails may be bigger than 180º.

If the radii of circles of circular arcs of opposite or all rails are identical, the rails located in the two relatively perpendicular planes may then be interconnected directly and adjoin each other. In a preferred embodiment the central angles of circular arcs will be exactly 180º and the rails in shape of a half-circle will be interconnected and thus located on a three-dimensional closed curve and form a compact unit. In a preferred embodiment of the joint with paired rails with identical radii of circles of circular arcs, it will basically be a single rail consisting of four segments in shape of a half-circle, i.e., partial rails. The rails, particularly if they are defined by central angles of circular arcs smaller than 180º, do not have to be interconnected directly. In such case they are interconnected by means of a rigid frame. Similarly, sliders may be interconnected by means of the rigid frame in an embodiment where rails suitable for connection to the first and the second relatively moving parts of the exoskeleton move relative to each other on paths defined by sliders.

There will be a frequent embodiment when only the circles of circular arcs of opposite rails will have the same radii, while rails perpendicular to each other will have different radii relative to each other.

In an even more preferred embodiment, all four rails are in the form of four half-circles, interconnected into a tree-dimensional closed shape such that the planes of arrangement of the adjacent half-circles form an angle of 90º.

In another preferred embodiment of the hollow joint according to the invention, the partial rails can be firmly interconnected by a straight frame, which may have the same profile as the rails. There may be two or three partial rails in this embodiment, out of which two are paired. Planes of arrangement of both partial rails in the embodiment with two rails, or a separate partial rail and two paired rails in the embodiment with three rails, form an angle of 90º. From one end or, in case the central angle of the circular arc of the unpaired rail is exactly 180º, from both ends of the unpaired rail in the straight direction the straight frame extends with length that equals the radii of circular arcs of the partial rails that are connected to the frame, thus the partial rail and the frame are located on a continuous three-dimensional curve and consequently form a compact unit. Each rail is provided with a slider and these sliders serve for connection to the first and the second relatively moving parts of the exoskeleton. This embodiment can be preferably used for joints, where a bend smaller than 180º is provided, namely from straightened position only in a single direction, e.g., for use in exoskeletons and rehabilitation devices for an elbow joint support.

The proposed joint design, together with variants of rail embodiments, allows for different methods of realization of slider movement on rail by means of drives allowing for transfer of force or torque, e.g., by an electric rotational or linear motor, piezo motor, fluid drive, etc. The drive allows to influence the relative movement of slider and rail. Slider movement may be sliding-translational or the slider may have a rotational guide and roll on the rail, e.g., by means of roller system, or in a preferred embodiment with the use of gear transmission, where both the slider or part thereof, e.g., a gear wheel, and the rail are provided with gearing. The embodiment with gearing allows for a simple implementation of joint fixation in a required position and accurate driving, more accurate than e.g., in the case of a friction/sliding contact. During the relative movement of the rail and slider the worm gear principle could be used. The slider and rail connection may also be of sliding nature and show a low friction coefficient. In such case the potential relative movement of parts connected by the joint must be provided by another mechanism. The drive, which is not located directly in contact with the rail, may be e.g., a system of ropes connected to the individual parts of the joint or the exoskeleton.

The hollow joint according to the invention may also be provided with a sensor or sensors scanning the movement of sliders on rails. The sensors may be of various shapes and use various principles. It is possible to use rotational sensors, e.g., potentiometers, Hall sensor, etc. It is also possible to implement linear or rounded sensors, e.g., magnetic tapes. Another suitable type may be incremental-optical sensors, etc. With the use of information obtained from the sensors it is possible to effectively control the drive providing the relative movement of the slider and the rail. In addition, based on scanning of the user's hand movement, it is possible to use the joint to control a robot.

The joint does not need a drive for use in passive exoskeletons or rehabilitation devices. On the contrary, a passive exoskeleton may resist by e.g., spring, friction, or another mechanism, and thus allow for exercising a respective human body part. Potentially, such embodiment may provide "support" e.g., of a limb under bigger load for operators at a production line.

Rails with slider may have, in the basic embodiment, relative free movement. The slider or the rail may be, in the preferred embodiment, provided with a fixation means, which fixes the slider in the given position on the rail and thus prevents movement in one or both axes. Such fixation means may be a locking, friction or another mechanism. This may be a solution for a purely positionable exoskeleton, which holds human body parts in the required angle.

Rails, or the exoskeleton connecting rails or sliders, may be detachable or separable into parts and connected by the joint, which allows for easier attachment of the exoskeleton with the joint to human body parts, the relative movement of which the joint should provide, e.g., attachment of the joint to the user's wrist. In order to better match the position of the hollow joint according to the invention, after fitting the exoskeleton, to the point of rotation of the human joint, e.g., user's wrist, the first and the second relatively moving parts of the exoskeleton, to which the joint is connected, may have a design that allows for their extension or retraction, may be e.g., telescopic, or equipped with movable sleeves. Such embodiment will be particularly preferable for the exoskeleton or rehabilitation device in the medical center, where the sizes of relatively moving parts of the exoskeleton have to be adjusted individually for each user. In case it is necessary to provide a larger inner space of the mechanical joint, bigger radii of circles of circular arcs of rails need to be provided. It is difficult to design a mechanism that would allow for dynamic change of the radii of circles of circular arcs of the rails. An alternative may be to replace entire rails, if necessary, e.g., from a set of standardized dimensions S, M, L, XL, etc.

Relatively moving parts of the joint, i.e., sliders and rails, or the exoskeleton, may be made of different construction materials, e.g., plastics of high hardness, light metal alloys, etc. The specific strength of the material depends on the required joint application, on the anticipated load of the joint during normal use, or on the required exoskeleton weight. In case of an exoskeleton which increases strength of the user, e.g., for handling heavy loads, the strength of the individual parts of the joint must be significantly higher, e.g., achieved by production thereof from light metal alloys, than in an exoskeleton for rehabilitation purposes, where hard plastic is sufficient. Potential rollers, gear transmissions, etc., can be produced by standard procedures.

Rails and other parts of the joint may be produced by various methods. Welding by simple laser-burned elements, additive technologies, such as 3D printing, etc., can be used. When plastics of high hardness are used, the component may be cast into molds and then milled. Production by injection molding is also possible and subsequent machining may not be necessary.

Further advantages of the present invention in combination with the previous include a possibility to transfer load on the human joint to the device structure, a possibility to drive the joint movement, e.g., for rehabilitation needs, or enhancing user's movement, a possibility to add mechanical stoppers in individual axes of movement for protection of the user's joint against excessive bending, and overall layout of the mechanism allowing for an embodiment of the exoskeleton as a "wearable" compact system.

The solution is particularly intended for use as the wrist joint of the exoskeleton; however, the same design principle can be used for other limb joints of the exoskeleton. This also made possible by the fact that the joint according to the invention provides mechanical guarantee of the stability of rotation axes, large inner space of the joint, where during the joint movement no collision of mechanical parts of the joint with the user's limb occurs even at maximum bending.

The joint according to the invention can be used in many joints of the exoskeleton or rehabilitation device, which in case of combined use of common rotational joints lead to a reduced maximum possible range of movement of the joint and to a structurally complicated solutions with many degrees of freedom, which in turn leads to more complicated control of the kinematics of the system. It is also possible to combine it with other mechanisms, e.g., with a solution of exoskeleton design for a palm (a glove with fingers for rehabilitation), elbow and shoulder, which provides necessary mobility.

Another object of the invention is also an exoskeleton increasing user's strength or a rehabilitation device for rehabilitation purposes, which are provided with at least one joint described above. The joint solution is particularly intended for use as a wrist joint of the exoskeleton, however, the same design principle can be used for other limb joints of the exoskeleton, e.g., for a hip, knee, ankle, etc.

### Description of Drawings

The summary of the invention is further described in exemplary embodiments that are described with reference to accompanying drawings, in which:
Figure 1 is an axonometric view illustrating an embodiment of a hollow joint according to this invention with two pairs of interconnected partial rails according to this invention for rehabilitation of wrist joint, where central angles of circular arcs of all partial rails have the size of 180º.
Figure 2 is a top view illustrating the same embodiment of the hollow joint according to this invention for rehabilitation of wrist joint.
Figure 3 is a top view illustrating the same embodiment of the hollow joint according to this invention for rehabilitation of wrist joint, with palm tilted to the right.
Figure 4 is a side view illustrating the same embodiment of the hollow joint according to this invention for rehabilitation of wrist joint, with palm of the hand tilted downwards.
Figure 5 is a side view illustrating the same embodiment of the hollow joint according to this invention for rehabilitation of wrist joint, with palm extending in the same direction as the forearm.
Figure 6 is an axonometric view illustrating an embodiment of a hollow joint with two interconnected partial rails according to this invention for rehabilitation of wrist joint, where central angles of circular arcs of both partial rails have the size of 180º and where the palm of the hand is tilted downwards.
Figure 7 is an axonometric view illustrating an embodiment of a hollow joint with two pairs of rails according to this invention for rehabilitation of wrist joint, where central angles of circular arcs of all rails have the size smaller than 180º and sliders are connected by a frame.
Figure 8 is a side view illustrating the embodiment of the hollow joint from Figure 7, with the palm of the hand tilted upwards.
Figure 9 is an axonometric view illustrating an embodiment of a hollow joint with two interconnected partial rails according to this invention for rehabilitation of an ankle, where central angles of circular arcs of both partial rails have the size of 180º.
Figure 10 is an axonometric view illustrating the embodiment of the hollow joint from Figure 9 with slightly pointed toes.
Figure 11 is an axonometric view illustrating an embodiment of a hollow joint with two interconnected partial rails according to this invention for rehabilitation of a hip joint, where the central angle of a circular arc of one of the partial rails has the size of 180º.
Figure 12 is an axonometric view illustrating an embodiment of a hollow joint with two interconnected partial rails according to this invention for rehabilitation of an elbow, where central angles of circular arcs of both partial rails have the size of 180º.

### Exemplary Embodiments of the Invention

The invention will be further described in exemplary embodiments with reference to respective drawings.

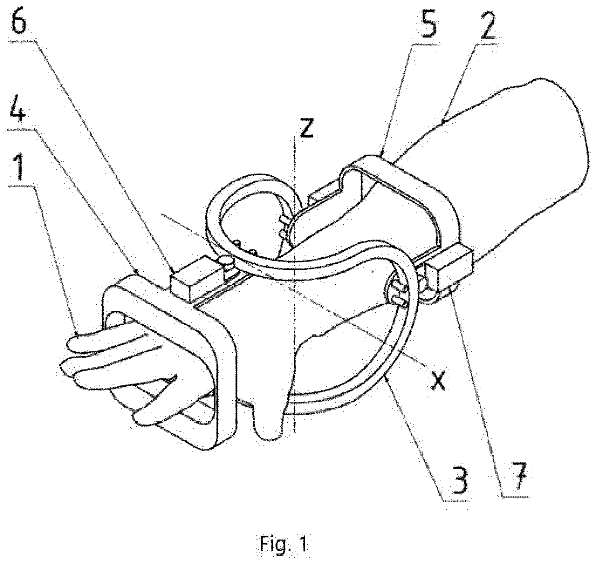

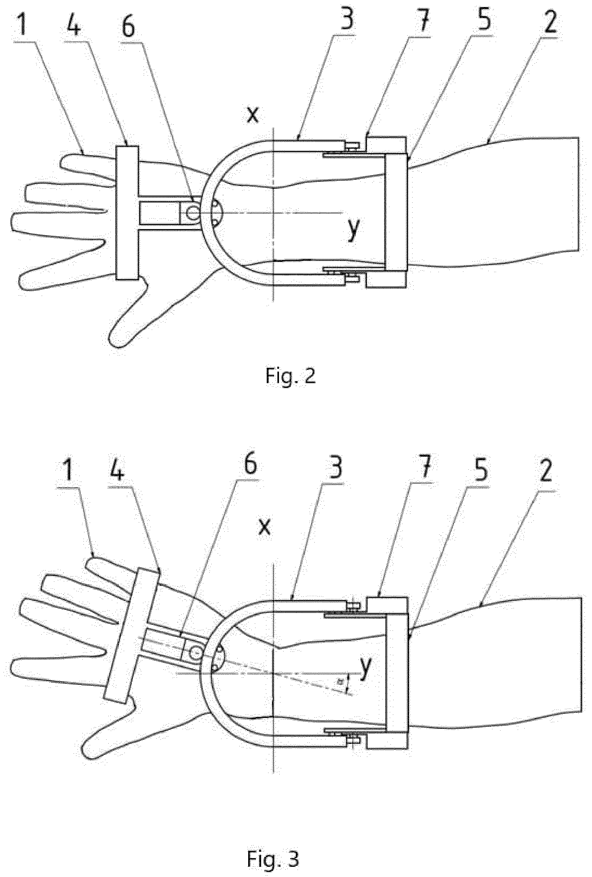

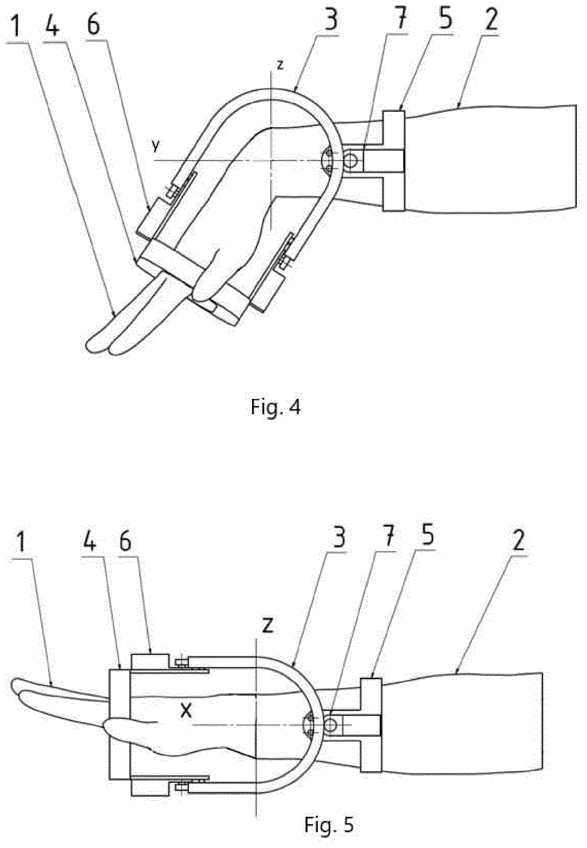

The first exemplary embodiment is a joint for use in exoskeletons and rehabilitation devices for supporting a wrist joint. An axonometric view illustrating the embodiment of this hollow joint with two pairs of interconnected rails is shown in Fig. 1. The joint connects a first and a second relative flexible parts **4**, **5** of the exoskeleton, where the first part **4** of the exoskeleton is connected to a first moving object **1**, i.e., before the wrist joint at the end of the limb in the area of palm or fingers, while the second part **5** of the exoskeleton is attached to a second moving object **2**, specifically behind the wrist joint of the limb on the forearm. Both parts **4**, **5** of the exoskeleton are in shape of a sleeve. On the first part **4** of the exoskeleton, two first sliders **6** are formed opposite to each other, while the second part **5** of the exoskeleton is provided with two opposite second sliders **7**. The joint according to this embodiment further includes a first, a second, a third and a fourth partial rail in shape of circular arcs, with the first and third partial rails located in two planes parallel to each other, wherein the centers of circles of circular arcs are located on a single straight line perpendicular to the respective planes defined by the circular arcs, and the circles of their circular arcs have the same radii, and the second and fourth partial rails are located in two planes parallel to each other, wherein the centers of circles of their circular arcs are located on a single straight line perpendicular to the respective planes defined by the circular arcs. On each partial rail, as shown in Fig. 1, one first or second slider **6**, **7** moves, which is provided with a set of rolling elements allowing for movement of the partial rail relative to the respective first or second part **4**, **5** of the exoskeleton. The radii of circles of circular arcs of the rails are identical and at the same time the central angles of circular arcs are exactly 180º. Planes of arrangement of the adjacent partial rails form a degree of 90º. Therefore, partial rails in shape of a half-circle are located on a three-dimensional closed curve and form a compact rail **3**. Fig. 2 is then a top view and Fig. 5 is a side view illustrating the same embodiment of the hollow joint according to the invention.

The disclosed hollow joint has two degrees of freedom, as shown in Figs. 3 and 4, when with the correct placement of the hollow joint on the wrist joint the axes of user's wrist movement are identical to the axes of the hollow joint movement. Figure 3 is a top view illustrating the same embodiment of the hollow joint for rehabilitation of the wrist joint, when the user's palm is tilted to the right by an angle α simultaneously with the movement of the first sliders **6** on the rail **3.** Figure 4 is a side view illustrating the same embodiment of the hollow joint according to this invention for rehabilitation of wrist joint, when the user's palm is tilted downwards simultaneously with the movement of the second sliders **7** on the rail **3.**

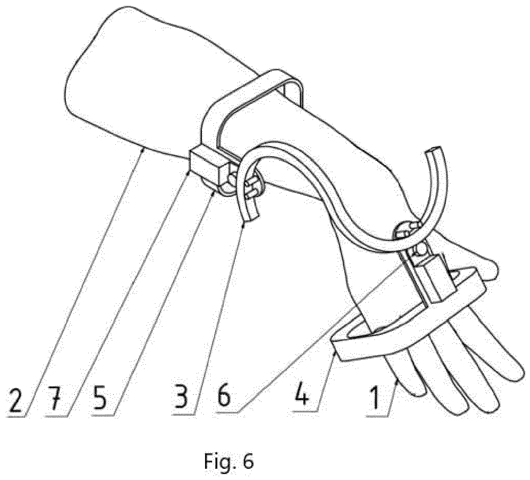

An alternative embodiment of the joint for use in exoskeletons and rehabilitation devices for support of the wrist joint is axonometrically illustrated in Fig. 6. This embodiment differs from the previous one in that there is one first slider **6** formed on the first part **4** of the exoskeleton and one second slider **7** is formed on the second part **5** of the exoskeleton, with each slider **6**, **7**, provided with a set of rolling elements allowing for movement of the partial rail relative to the respective first or second part **4**, **5** of the exoskeleton. There are only two partial rails in this embodiment, where the planes of arrangement of the adjacent partial rails form an angle of 90º, the radii of circles of circular arcs of partial rails are identical and at the same time the central angles of circular arcs are exactly 180º. The partial rails in shape of a half-circle are thus located on a three-dimensional curve and form a compact rail **3.** Due to the movement of the second slider **6** on the rail **3**, the user's palm is tilted downwards.

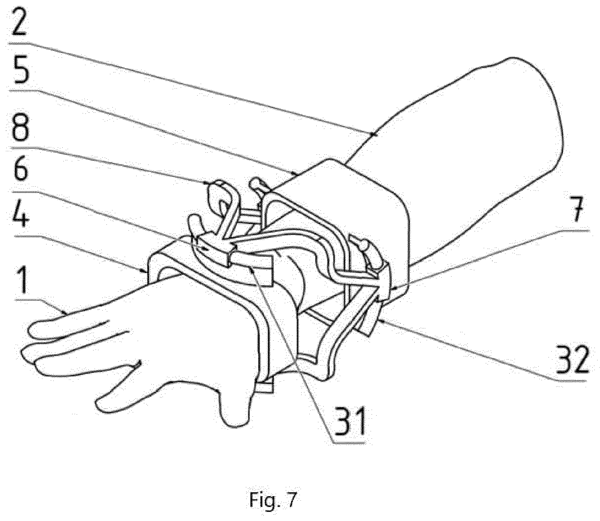

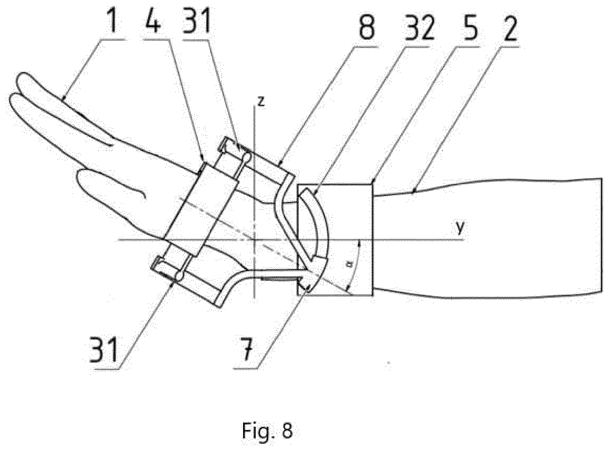

Figure 7 is an axonometric view illustrating further alternative embodiment of the joint for use in exoskeletons and rehabilitation devices for support of the wrist joint. The joint connects the first and the second relatively moving part 4, 5 of the exoskeleton in form of sleeves. On the first part **4** of the exoskeleton there are two first rails **31** formed opposite to each other, while the second part **5** of the exoskeleton is provided with two opposite second rails **32**. These first and second rails **31** and **32** are in shape of circular arcs, where both first rails **31** are located in two planes parallel to each other, wherein the centers of circles of their circular arcs are located on a single straight line perpendicular to the respective planes defined by the circular arcs and the circles of their circular arcs have the same radii, and both second rails **32** are located in two planes parallel to each other, wherein the centers of circles of their circular arcs are located on a single straight line perpendicular to the respective planes defined by the circular arcs. One first or second slider **6**, **7** moves on each partial rail, wherein all sliders **6**, **7** are connected by a frame **8**. The central angles of circular arcs of first and second rails **31** and **32** are smaller than 180º, thereby limiting the range of movement of the joint, and the planes of arrangement of first and second rails **31** and **32** form an angle of 90º. Figure 8 is then a side view illustrating the embodiment of the hollow joint according to this invention for rehabilitation of the wrist joint, when simultaneously with the movement of second sliders **7** on second rails **32**, the user's palm of the hand is tilted upwards by an angle α.

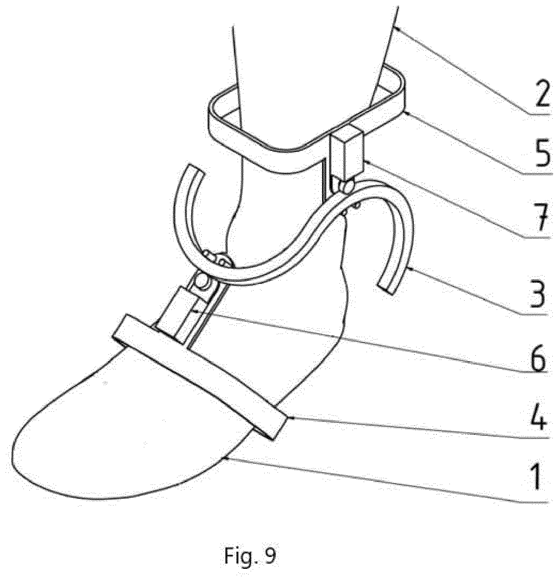

The fourth exemplary embodiment is a joint for use in exoskeletons and rehabilitation devices for support of an ankle joint. An axonometric view illustrating the embodiment of this hollow joint with two interconnected partial rails is shown in Fig. 9. The joint connects the first and the second relatively moving part **4**, **5** of the exoskeleton, with the first part **4** of the exoskeleton connected to a first moveable object **1**, i.e., before the ankle at the end of the limb in the area of foot or toes, while the second part **5** of the exoskeleton is attached to a second moveable object **2**, i.e., specifically on calf behind the ankle. Both parts **4**, **5** of the exoskeleton are in shape of a sleeve. There are only two partial rails in this embodiment, the planes of arrangement of the adjacent partial rails form an angle of 90º, the radii of circles of circular arcs of partial rails are identical and at the same time the central angles of circular arcs are exactly 180º. Partial rails in shape of a half-circle are thus located on a three-dimensional curve and form a compact rail **3**. One first slider **6** is formed on the first part **4** of the exoskeleton and one second slider **7** is formed on the second part **5** of the exoskeleton, with each slider **6**, **7** provided with a set of rolling elements allowing for movement of a respective partial rail relative to the respective first or second parts **4**, **5** of the exoskeleton.

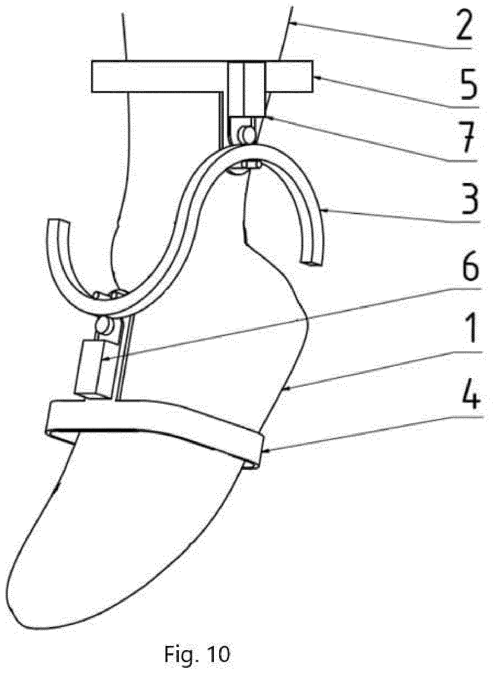

Figure 10 is an axonometric view illustrating the embodiment of the hollow joint from Figure 9 with slightly pointed toes, which is achieved by movement of the second slider **7** on the rail **3**.

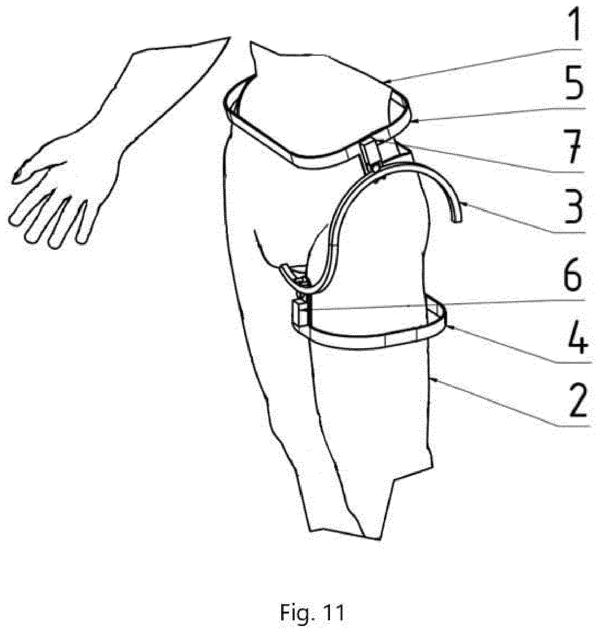

The fifth embodiment example is a joint for use in exoskeletons and rehabilitation devices for support of an ankle joint. An axonometric view illustrating the embodiment of this hollow joint with two interconnected partial rails is shown in Fig. 11. The joint connects the first and the second relatively moving part **4**, **5** of the exoskeleton, with the first part **4** of the exoskeleton connected to a first moveable object **1**, in this case a thigh, while the second part **5** of the exoskeleton is attached to a second moveable object **2**, specifically to a torso, e.g., in the area of waist. Both parts **4**, **5** of the exoskeleton are in shape of a sleeve. There are only two partial rails in this embodiment, the planes of arrangement of the adjacent partial rails form an angle of 90º and the radii of circles of circular arcs of the partial rails are identical. The central angles of circular arcs are of different sizes, the central angle at the partial rail closer to the torso at the side is exactly 180º, the central angle at the partial rail closer to the thigh is smaller, since a side leg lift does not require such big movement range. The partial rails in shape of a half-circle are thus located on a three-dimensional curve and form a compact rail **3**. One first slider **6** is formed on the first part **4** of the exoskeleton and one second slider **7** is formed on the second part **5** of the exoskeleton, with each slider **6**, **7** provided with a set of rolling elements allowing for movement of the respective partial rail relative to the respective first or second part **4**, **5** of the exoskeleton.

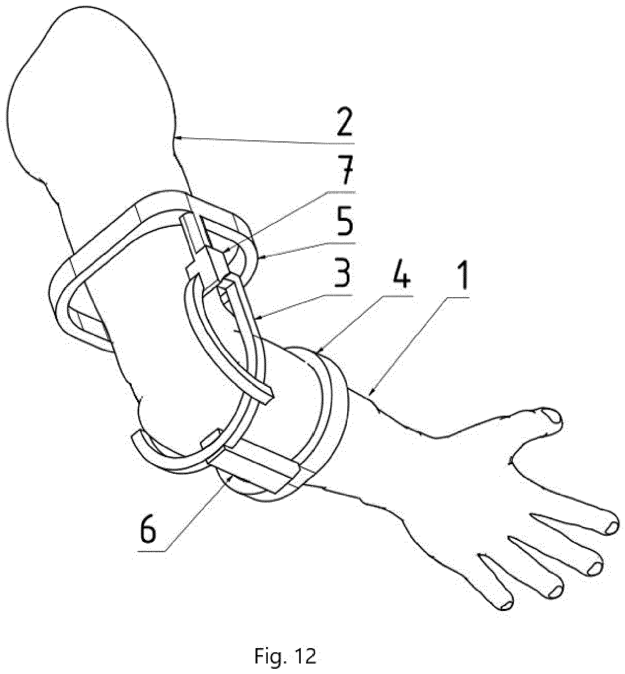

The sixth exemplary embodiment is a joint for use in exoskeletons and rehabilitation devices for support of an elbow joint. An axonometric view illustrating the embodiment of this hollow joint with two firmly interconnected partial rails is shown in Fig. 12. There are only two partial rails in this embodiment, where the planes of arrangement of both partial rails form an angle of 90º, the radii of circles of circular arcs of partial rails are identical and at the same time the central angles of circular arcs are exactly 180º. The partial rails in shape of a half-circle are connected by a short straight frame with the length of the radii of circular arcs and of the same profile as the rails, so that both partial rails and the frame are located on a continuous three-dimensional curve and consequently form a compact unit. One first slider **6** is formed on the first part **4** of the exoskeleton and one second slider **7** is formed on the second part **5** of the exoskeleton, with each slider **6**, **7** provided with a set of rolling elements allowing for movement of the respective partial rail relative to the respective first or second part **4**, **5** of the exoskeleton. This joint interconnects the first and the second relatively moving part **4**, **5** of the exoskeleton, with the first part **4** of the exoskeleton connected to a first moveable object **1**, i.e., to a forearm, while the second part **5** of the exoskeleton is attached to a second moveable object **2**, specifically to the upper arm. Both parts **4**, **5** of the exoskeleton are in shape of a sleeve.

### Industrial Applicability

The present invention of an exoskeleton joint can be used for a construction of more compact wearable exoskeletons, e.g., for increasing user's movement strength, their protection or rehabilitation, while ensuring free movement of the user.

The design principle can be used in many exoskeleton joints, which in case of use of combinations of standard rotational joints lead to structurally complex solutions with many degrees of freedom, which lead to more complicated control of the system kinematics.

### List of Reference Numbers

- 1: First moving object
- 2: Second moving object
- 3: Rail
- 31: First and third rail
- 32: Second and fourth rail
- 4: First part of exoskeleton
- 5: Second part of exoskeleton
- 6: First slider
- 7: Second slider
- 8: Frame

## Claims

1. A joint with two degrees of freedom for use in exoskeletons and rehabilitation devices for connection of a first and a second relatively moving parts of the exoskeleton, the joint comprises at least a first and a second rail (**3, 31, 32**) in shape of circular arcs, which are located in two planes parallel to each other, wherein the centers of circles of the circular arcs differ, and further a movably arranged slider on each rail (**3, 31, 32**), **characterized in that** either the rails (**3, 31, 32**) are firmly interconnected and sliders (**6, 7**) are each suitable for connection to another relatively moving part of the exoskeleton, or the sliders (**6, 7**) are firmly interconnected and the rails (**3, 31, 32**) are each suitable for connection to another relatively moving part of the exoskeleton.

2. The joint according to claim 1, **characterized in that,** in addition to the first and the second rail (**3, 31, 32**) in shape of circular arcs, it comprises a parallel third and fourth rails (**3, 31, 32**), the first and the third rail (**31**) are located in two planes parallel to each other, wherein the centers of circles of their circular arcs are located on a single straight line perpendicular to the respective planes defined by the circular arcs and the circles of circular arcs have the same radius, and the second and the fourth rails (**32**) are located in two planes parallel to each other, wherein the centers of circles of their circular arcs are located on a single straight line perpendicular to the respective planes defined by the circular arcs, and the third and fourth rails (**3, 31, 32**) are provided with another slider (**6, 7**) moveable thereon.

3. The joint according to claim 1 or 2, **characterized in that** the radii of circles of circular arcs of rails (**3, 31, 32**) are identical.

4. The joint according to any of preceding claims, **characterized in that** either the rails (**3, 31, 32**) are firmly interconnected or the sliders (**6, 7**) are firmly interconnected by a frame (**8**).

5. The joint according to claim 3, **characterized in that** the central angles of circular arcs are exactly 180º and the rails (**3, 31, 32**) in shape of a half-circle are located on a three-dimensional closed curve and form a compact unit.

6. The joint according to any of preceding claims, **characterized in that** the straight lines intersecting centers of circles of circular arcs and perpendicular to the respective planes defined by the circular arcs intersect each other in a single point.

7. Rehabilitation device provided with the joint according to any of preceding claims.

## Patentansprüche

1. Gelenk mit zwei Freiheitsgraden zur Verwendung in Exoskeletten und Rehabilitationsvorrichtungen zur Verbindung von ersten und zweiten relativ beweglichen Teilen des Exoskeletts, wobei das Gelenk zumindest eine erste und eine zweite Schiene **(3, 31, 32)** in Form von Kreisbögen, die in zwei zueinander parallelen Ebenen liegen, wobei die Mittelpunkte von Kreisen der Kreisbögen unterschiedlich sind, und ferner einen beweglich angeordneten Schieber auf jeder Schiene **(3, 31, 32)** umfasst, **dadurch gekennzeichnet, dass** entweder die Schienen **(3, 31, 32)** fest miteinander verbunden sind und Schieber **(6, 7)** jeweils zur Verbindung mit einem anderen relativ beweglichen Teil des Exoskeletts geeignet sind, oder die Schieber **(6, 7)** fest miteinander verbunden sind und die Schienen **(3, 31, 32)** jeweils zur Verbindung mit einem anderen relativ beweglichen Teil des Exoskeletts geeignet sind.

2. Das Gelenk nach Anspruch 1, **dadurch gekennzeichnet, dass** es zusätzlich zu der ersten und der zweiten Schiene **(3, 31, 32)** in Form von Kreisbögen parallele dritte und vierte Schienen **(3, 31, 32)** umfasst, wobei die erste und die dritte Schiene **(31)** in zwei zueinander parallelen Ebenen angeordnet sind, die Mittelpunkte von Kreisen ihrer Kreisbögen auf einer einzigen geraden Linie senkrecht zu den jeweiligen durch die Kreisbögen definierten Ebenen liegen und die Kreise der Kreisbögen den gleichen Radius haben, und die zweiten und die vierten Schienen **(32)** in zwei zueinander parallelen Ebenen angeordnet sind, wobei die Mittelpunkte von Kreisen ihrer Kreisbögen auf einer einzigen geraden Linie senkrecht zu den jeweiligen durch die Kreisbögen definierten Ebenen liegen, und die dritten und vierten Schienen **(3, 31, 32)** mit einem weiteren darauf beweglichen Schieber **(6, 7)** versehen sind.

3. Das Gelenk nach Anspruch 1 oder 2, **dadurch gekennzeichnet, dass** die Radien von Kreisen der Kreisbögen der Schienen **(3, 31, 32)** identisch sind.

4. Das Gelenk nach irgendeinem der vorhergehenden Ansprüche, **dadurch gekennzeichnet, dass** entweder die Schienen **(3, 31, 32)** fest miteinander verbunden sind oder die Schieber **(6, 7)** durch einen Rahmen **(8)** fest miteinander verbunden sind.

5. Das Gelenk nach Anspruch 3, **dadurch gekennzeichnet, dass** die zentralen Winkel der Kreisbögen genau 180° betragen und die Schienen **(3, 31, 32)** in Form eines Halbkreises auf einer dreidimensionalen geschlossenen Kurve liegen und eine kompakte Einheit bilden.

6. Das Gelenk nach irgendeinem der vorhergehenden Ansprüche, **dadurch gekennzeichnet, dass** sich die geraden Linien, die Mittelpunkte von Kreisen der Kreisbögen schneiden und senkrecht zu den jeweiligen durch die Kreisbögen definierten Ebenen verlaufen, in einem einzigen Punkt schneiden.

7. Rehabilitationsvorrichtung bereitgestellt mit dem Gelenk nach irgendeinem der vorhergehenden Ansprüche.

## Revendications

1. - Articulation à deux degrés de liberté destinée à être utilisée dans des exosquelettes et des dispositifs de rééducation pour la liaison de première et seconde parties de l'exosquelette mobiles l'une relativement à l'autre, l'articulation comprend au moins un premier et un deuxième rail (3, 31, 32) en forme d'arcs circulaires, qui sont situés dans deux plans parallèles l'un à l'autre, les centres de cercles des arcs circulaires étant différents, et en outre un coulisseau disposé de manière mobile sur chaque rail (3, 31, 32), **caractérisée par le fait que** soit les rails (3, 31, 32) sont fermement reliés l'un à l'autre et les coulisseaux (6, 7) sont chacun appropriés pour une liaison à une autre partie relativement mobile de l'exosquelette, soit les coulisseaux (6, 7) sont fermement reliés l'un à l'autre et les rails (3, 31, 32) sont chacun appropriés pour une liaison à une autre partie relativement mobile de l'exosquelette.

2. - Articulation selon la revendication 1, **caractérisée par le fait que**, en plus du premier et du deuxième rail (3, 31, 32) en forme d'arcs circulaires, elle comprend des troisième et quatrième rails parallèles (3, 31, 32), les premier et troisième rails (31) sont situés dans deux plans parallèles l'un à l'autre, les centres de cercles de leurs arcs circulaires étant situés sur une seule ligne droite perpendiculaire aux plans respectifs définis par les arcs circulaires et les cercles des arcs circulaires ayant le même rayon, et les deuxième et quatrième rails (32) sont situés dans deux plans parallèles l'un à l'autre, les centres de cercles de leurs arcs circulaires étant situés sur une seule ligne droite perpendiculaire aux plans respectifs définis par les arcs circulaires, et les troisième et quatrième rails (3, 31, 32) étant munis d'un autre coulisseau (6, 7) mobile sur ceux-ci.

3. - Articulation selon la revendication 1 ou 2, **caractérisée par le fait que** les rayons de cercles des arcs circulaires des rails (3, 31, 32) sont identiques.

4. - Articulation selon l'une quelconque des revendications précédentes, **caractérisée par le fait que** soit les rails (3, 31, 32) sont fermement reliés l'un à l'autre, soit les coulisseaux (6, 7) sont fermement reliés l'un à l'autre par un cadre (8).

5. - Articulation selon la revendication 3, **caractérisée par le fait que** les angles centraux des arcs circulaires sont exactement de 180° et les rails (3, 31, 32) en forme de demi-cercle sont situés sur une courbe fermée tridimensionnelle et forment une unité compacte.

6. - Articulation selon l'une quelconque des revendications précédentes, **caractérisée par le fait que** les lignes droites coupant les centres de cercles des arcs circulaires et perpendiculaires aux plans respectifs définis par les arcs circulaires se coupent en un seul point.

7. - Dispositif de rééducation muni de l'articulation selon l'une quelconque des revendications précédentes.
